# EUROPEAN PATENT APPLICATION

(11) **EP 2 127 672 A1**
(43) Date of publication of application: **02.12.2009**
(21) Application number: 07860318.0
(22) Date of filing: 27.12.2007
(51) Int. Cl.: A61K 45/00, A61K 38/00, A61K 39/395, A61P 31/22, A61P 43/00, C07K 14/705, C07K 16/28, C12N 5/10

(54) **INHIBITOR OF HERPESVIRUS INFECTION, METHOD FOR INHIBITION OF HERPESVIRUS INFECTION, AND USE THEREOF**

(30) Priority: 12.01.2007 JP 2007005229
(71) Applicant: Osaka University, Suita-shi Osaka 565-0871 (JP)
(72) Inventor: ARASE, Hisashi, 565-0871 Osaka (JP); SATOH, Takeshi, 565-0871 Osaka (JP); KAWAGUCHI, Yasushi, 4-6-1 Shirokanedai Minato-ku 108-8639 Tokyo (JP)
(74) Representative: Graf von Stosch, Andreas
(86) International application number: PCT/JP2007/075096
(87) International publication number: WO 2008/084710

(57) **Abstract**

The herpes virus infection inhibitor of the present invention contains an effective component capable of binding to glycoprotein B or a receptor for glycoprotein B and capable of inhibiting interaction between glycoprotein B and the receptor for glycoprotein B. This provides a herpes virus infection inhibitor, a method for inhibiting infection with herpes virus, and representative use thereof.

### Sequence Listing Free Text

SEQUENCE LISTING

<110> Osaka University

<120> Herpes virus inhibitor, herpes virus inhibitory method and use thereof

<130> G20070060

<150> JP 2007-005229
<151> 2007-01-12

<160> 11

<170> PatentIn Ver. 2.1

<210> 1
<211> 903
<212> PRT
<213> human herpesvirus 1

<400> 1
Met Arg Gln Gly Ala Ala Arg Gly Cys Arg Trp Phe Val Val Trp Ala
  1               5                  10                  15

Leu Leu Gly Leu Thr Leu Gly Val Leu Val Ala Ser Ala Ala Pro Ser
             20                  25                  30

Ser Pro Gly Thr Pro Gly Val Ala Ala Ala Thr Gln Ala Ala Asn Gly
         35                  40                  45

Gly Pro Ala Thr Pro Ala Pro Pro Ala Pro Gly Pro Ala Pro Thr Gly
     50                  55                  60

Asp Thr Lys Pro Lys Lys Asn Lys Lys Pro Lys Asn Pro Pro Pro Pro
 65                  70                  75                  80

Arg Pro Ala Gly Asp Asn Ala Thr Val Ala Ala Gly His Ala Thr Leu
                 85                  90                  95

Arg Glu His Leu Arg Asp Ile Lys Ala Glu Asn Thr Asp Ala Asn Phe
            100                 105                 110

Tyr Val Cys Pro Pro Pro Thr Gly Ala Thr Val Val Gln Phe Glu Gln
        115                 120                 125

Pro Arg Arg Cys Pro Thr Arg Pro Glu Gly Gln Asn Tyr Thr Glu Gly
    130                 135                 140

Ile Ala Val Val Phe Lys Glu Asn Ile Ala Pro Tyr Lys Phe Lys Ala

145                 150                 155                 160

Thr Met Tyr Tyr Lys Asp Val Thr Val Ser Gln Val Trp Phe Gly His
                165                 170                 175

Arg Tyr Ser Gln Phe Met Gly Ile Phe Glu Asp Arg Ala Pro Val Pro
            180                 185                 190

Phe Glu Glu Val Ile Asp Lys Ile Asn Ala Lys Gly Val Cys Arg Ser
        195                 200                 205

Thr Ala Lys Tyr Val Arg Asn Asn Leu Glu Thr Thr Ala Phe His Arg
    210                 215                 220

Asp Asp His Glu Thr Asp Met Glu Leu Lys Pro Ala Asn Ala Ala Thr
225                 230                 235                 240

Arg Thr Ser Arg Gly Trp His Thr Thr Asp Leu Lys Tyr Asn Pro Ser
                245                 250                 255

Arg Val Glu Ala Phe His Arg Tyr Gly Thr Thr Val Asn Cys Ile Val
            260                 265                 270

Glu Glu Val Asp Ala Arg Ser Val Tyr Pro Tyr Asp Glu Phe Val Leu
        275                 280                 285

Ala Thr Gly Asp Phe Val Tyr Met Ser Pro Phe Tyr Gly Tyr Arg Glu
    290                 295                 300

Gly Ser His Thr Glu His Thr Ser Tyr Ala Ala Asp Arg Phe Lys Gln
305                 310                 315                 320

Val Asp Gly Phe Tyr Ala Arg Asp Leu Thr Thr Lys Ala Arg Ala Thr
                325                 330                 335

Ala Pro Thr Thr Arg Asn Leu Leu Thr Thr Pro Lys Phe Thr Val Ala
            340                 345                 350

Trp Asp Trp Val Pro Lys Arg Pro Ser Val Cys Thr Met Thr Lys Trp
        355                 360                 365

Gln Glu Val Asp Glu Met Leu Arg Ser Glu Tyr Gly Gly Ser Phe Arg
    370                 375                 380

Phe Ser Ser Asp Ala Ile Ser Thr Thr Phe Thr Thr Asn Leu Thr Glu
385                 390                 395                 400

Tyr Pro Leu Ser Arg Val Asp Leu Gly Asp Cys Ile Gly Lys Asp Ala
                405                 410                 415

Arg Asp Ala Met Asp Arg Ile Phe Ala Arg Arg Tyr Asn Ala Thr His
            420                 425                 430

Ile Lys Val Gly Gln Pro Gln Tyr Tyr Leu Ala Asn Gly Gly Phe Leu
        435                 440                 445

Ile Ala Tyr Gln Pro Leu Leu Ser Asn Thr Leu Ala Glu Leu Tyr Val
    450                 455                 460

Arg Glu His Leu Arg Glu Gln Ser Arg Lys Pro Pro Asn Pro Thr Pro
465                 470                 475                 480

Pro Pro Pro Gly Ala Ser Ala Asn Ala Ser Val Glu Arg Ile Lys Thr
                485                 490                 495

Thr Ser Ser Ile Glu Phe Ala Arg Leu Gln Phe Thr Tyr Asn His Ile
            500                 505                 510

Gln Arg His Val Asn Asp Met Leu Gly Arg Val Ala Ile Ala Trp Cys
        515                 520                 525

Glu Leu Gln Asn His Glu Leu Thr Leu Trp Asn Glu Ala Arg Lys Leu
    530                 535                 540

Asn Pro Asn Ala Ile Ala Ser Ala Thr Val Gly Arg Arg Val Ser Ala
545                 550                 555                 560

Arg Met Leu Gly Asp Val Met Ala Val Ser Thr Cys Val Pro Val Ala
                565                 570                 575

Ala Asp Asn Val Ile Val Gln Asn Ser Met Arg Ile Ser Ser Arg Pro
            580                 585                 590

Gly Ala Cys Tyr Ser Arg Pro Leu Val Ser Phe Arg Tyr Glu Asp Gln
        595                 600                 605

Gly Pro Leu Val Glu Gly Gln Leu Gly Glu Asn Asn Glu Leu Arg Leu
    610                 615                 620

Thr Arg Asp Ala Ile Glu Pro Cys Thr Val Gly His Arg Arg Tyr Phe
625                 630                 635                 640

Thr Phe Gly Gly Gly Tyr Val Tyr Phe Glu Glu Tyr Ala Tyr Ser His
                645                 650                 655

Gln Leu Ser Arg Ala Asp Ile Thr Thr Val Ser Thr Phe Ile Asp Leu
            660                 665                 670

Asn Ile Thr Met Leu Glu Asp His Glu Phe Val Pro Leu Glu Val Tyr
        675                 680                 685

Thr Arg His Glu Ile Lys Asp Ser Gly Leu Leu Asp Tyr Thr Glu Val
    690                 695                 700

Gln Arg Arg Asn Gln Leu His Asp Leu Arg Phe Ala Asp Ile Asp Thr
705                 710                 715                 720

Val Ile His Ala Asp Ala Asn Ala Ala Met Phe Ala Gly Leu Gly Ala
                725                 730                 735

Phe Phe Glu Gly Met Gly Asp Leu Gly Arg Ala Val Gly Lys Val Val
            740                 745                 750

Met Gly Ile Val Gly Gly Val Val Ser Ala Val Ser Gly Val Ser Ser
        755                 760                 765

Phe Met Ser Asn Pro Phe Gly Ala Leu Ala Val Gly Leu Leu Val Leu
    770                 775                 780

Ala Gly Leu Ala Ala Ala Phe Phe Ala Phe Arg Tyr Val Met Arg Leu
785                 790                 795                 800

Gln Ser Asn Pro Met Lys Ala Leu Tyr Pro Leu Thr Thr Lys Glu Leu
                805                 810                 815

Lys Asn Pro Thr Asn Pro Asp Ala Ser Gly Glu Gly Glu Glu Gly Gly
            820                 825                 830

Asp Phe Asp Glu Ala Lys Leu Ala Glu Ala Arg Glu Met Ile Arg Tyr
        835                 840                 845

Met Ala Leu Val Ser Ala Met Glu Arg Thr Glu His Lys Ala Lys Lys
    850                 855                 860

Lys Gly Thr Ser Ala Leu Leu Ser Ala Lys Val Thr Asp Met Val Met
865                 870                 875                 880

Arg Lys Arg Arg Asn Thr Asn Tyr Thr Gln Val Pro Asn Lys Asp Gly
                885                 890                 895

Asp Ala Asp Glu Asp Asp Leu
            900

<210> 2
<211> 27
<212> DNA

<213> Artificial Sequence

<220>
<223> Description of Artificial Sequence:Artificially
      synthesized primer sequence

<400> 2
aatgaattca acaaggccat gggtcgg                                          27


<210> 3
<211> 34
<212> DNA
<213> Artificial Sequence

<220>
<223> Description of Artificial Sequence:Artificially
      synthesized primer sequence

<400> 3
aataatgcgg ccgcagggct gtccattggt tagg                                  34


<210> 4
<211> 29
<212> DNA
<213> Artificial Sequence

<220>
<223> Description of Artificial Sequence:Artificially
      synthesized primer sequence

<400> 4
aatctcgagc agcctagtgg ctccacagg                                        29


<210> 5
<211> 30
<212> DNA
<213> Artificial Sequence

<220>
<223> Description of Artificial Sequence:Artificially
      synthesized primer sequence

<400> 5
aataatctcg agaaccctga tggcagtgtc                                       30

<210> 6
<211> 31
<212> DNA
<213> Artificial Sequence

<220>
<223> Description of Artificial Sequence:Artificially
      synthesized primer sequence

<400> 6
aatctcgagg cggctccgag ttcccccggc a                                    31


<210> 7
<211> 29
<212> DNA
<213> Artificial Sequence

<220>
<223> Description of Artificial Sequence:Artificially
      synthesized primer sequence

<400> 7
aatgaattcc gtgtcgatgt cggcgaagc                                       29


<210> 8
<211> 29
<212> DNA
<213> Artificial Sequence

<220>
<223> Description of Artificial Sequence:Artificially
      synthesized primer sequence

<400> 8
aatctcgaga aatatgcctt ggtggatgc                                       29


<210> 9
<211> 29
<212> DNA
<213> Artificial Sequence

<220>
<223> Description of Artificial Sequence:Artificially
      synthesized primer sequence

<400> 9

aatctcgagc atgttgttcg gggtggccg                                    29

<210> 10
<211> 303
<212> PRT
<213> Human

<400> 10
Met Gly Arg Pro Leu Leu Leu Pro Leu Leu Pro Leu Leu Leu Pro Pro
1               5                   10                  15

Ala Phe Leu Gln Pro Ser Gly Ser Thr Gly Ser Gly Pro Ser Tyr Leu
            20                  25                  30

Tyr Gly Val Thr Gln Pro Lys His Leu Ser Ala Ser Met Gly Gly Ser
        35                  40                  45

Val Glu Ile Pro Phe Ser Phe Tyr Tyr Pro Trp Glu Leu Ala Thr Ala
    50                  55                  60

Pro Asp Val Arg Ile Ser Trp Arg Arg Gly His Phe His Gly Gln Ser
65                  70                  75                  80

Phe Tyr Ser Thr Arg Pro Pro Ser Ile His Lys Asp Tyr Val Asn Arg
                85                  90                  95

Leu Phe Leu Asn Trp Thr Glu Gly Gln Lys Ser Gly Phe Leu Arg Ile
            100                 105                 110

Ser Asn Leu Gln Lys Gln Asp Gln Ser Val Tyr Phe Cys Arg Val Glu
        115                 120                 125

Leu Asp Thr Arg Ser Ser Gly Arg Gln Gln Trp Gln Ser Ile Glu Gly
    130                 135                 140

Thr Lys Leu Ser Ile Thr Gln Ala Val Thr Thr Thr Thr Gln Arg Pro
145                 150                 155                 160

Ser Ser Met Thr Thr Thr Trp Arg Leu Ser Ser Thr Thr Thr Thr Thr
                165                 170                 175

Gly Leu Arg Val Thr Gln Gly Lys Arg Arg Ser Asp Ser Trp His Ile
            180                 185                 190

Ser Leu Glu Thr Ala Val Gly Val Ala Val Ala Val Thr Val Leu Gly
        195                 200                 205

Ile Met Ile Leu Gly Leu Ile Cys Leu Leu Arg Trp Arg Arg Arg Lys

210                 215                 220

Gly Gln Gln Arg Thr Lys Ala Thr Thr Pro Ala Arg Glu Pro Phe Gln
225                 230                 235                 240

Asn Thr Glu Glu Pro Tyr Glu Asn Ile Arg Asn Glu Gly Gln Asn Thr
                245                 250                 255

Asp Pro Lys Leu Asn Pro Lys Asp Asp Gly Ile Val Tyr Ala Ser Leu
            260                 265                 270

Ala Leu Ser Ser Ser Thr Ser Pro Lys Ala Pro Pro Ser His Arg Pro
        275                 280                 285

Leu Lys Ser Pro Gln Asn Glu Thr Leu Tyr Ser Val Leu Lys Ala
    290                 295                 300


<210> 11
<211> 434
<212> PRT
<213> Artificial Sequence

<220>
<223> Description of Artificial Sequence:Artificially
      synthesized protein sequence

<400> 11

## Description

### Technical Field

The present invention relates to a herpes virus infection inhibitor, a method for inhibiting infection with herpes virus, and use thereof.

### Background Art

Herpes virus is a family of animal DNA viruses, and is classified into Herpes simplex virus, Cytomegalovirus, Epstein-Barr virus etc. Herpes simplex virus is classified into HSV-1 (Herpes simplex virus type 1) and HSV-2 (Herpes simplex virus type 2). HSV-1 infects a human via skin or mucous membrane, latently infects a spinal ganglion or a trigeminal ganglion, and causes keratitis, herpes simplex encephalitis etc. HSV-2 causes genital herpes, neonatal herpes, herpes meningitis etc., and latently infects spinal ganglion.

Medical expense for infection with herpes simplex virus costs 350,000,000,000 yen. As a remedy for the infection, acyclovir that is an antiviral medicine is frequently used. Acyclovir inhibits only the duplication of DNA in herpes virus-infected cells and therefore causes cytotoxicity only in the infected cells.

On the other hand, it has been clarified that cells related to immune reaction (immune cells) such as T cells, B cells, NK cells, dendritic cells, macrophages, mastcells, basophils, eosinophils, and neutrophils exhibit expression of pair receptors including an activating receptor and an inhibitory receptor that are molecules controlling activation of the cells, and these receptors play a significant role in controlling the immune reaction. Although the activating receptor and the inhibitory receptor have high homology in an extracellular region, the receptors have functions contradicting each other (activation and inhibition).

The activating receptor binds to a cell expressing its ligand, and activates immune cells. Activating immune cells indicates that production of cytokine from immune cells and cytotoxicity are induced. The activating receptor has positively charged amino acids in its transmembrane region. The activating receptor binds to adaptor molecules including ITAM (Immunoreceptor Tyrosine-based Activation Motif) sequence [Y-x-x-L-x(6-8)-Y-x-x-x-L (x is any amino acid) such as DAP12, CD3z and FcRv via the positively charged amino acids. Thus, the activating receptor transmits an activation signal.

On the other hand, the inhibitory receptor includes ITIM (Immunoreceptor Tyrosine-based Inhibitory Motif) sequence [(I/V/L/S)-x-Y-x-x-(L/V) (x is any amino acid) and blocks a signal from the activating receptor by use of phosphatase such as SHP-1. Thus, the inhibitory receptor inhibits activation of immune cells.

A known example of the pair receptors is PILR (Paired Ig-like Type 2 Receptor). PILR is a pair receptor including a PILRα that is an inhibitory receptor and a mouse PILRβ that is an activating receptor. PILR is widely expressed in immune cells such as NK cells, dendritic cells, macrophages, and mast cells.

PILRα (sometimes referred to as FDF03) is cloned both in cases of humans and mice (Non-Patent Literatures 1 and 2). Further, the inventors of the present invention carried out cloning of a mouse PILR-L (PILR Ligand) that is a ligand of a mouse PILRα and PILRβ in Non-Patent Literature 3. In Non-Patent Literature 3, the inventors demonstrated in Non-Patent Literature 3 that a signal via binding of PILR-L and PILRβ activates NK cells and dendritic cells, and that PILRα of a mouse specifically recognizes CD99 of the mouse and contributes to regulation of immune reaction.

On the other hand, it is considered that some of latently infecting viruses express a ligand for the inhibitory receptor (Non-Patent Literature 4).

### Citation List

Non-Patent Literature 1
   Mousseau D. D., et al., J. Biol. Chem. 275 (2000) p.4467-4474
Non-Patent Literature 2
   Fournier, N., et al., J. Immunol. 165 (2000) p.1197-209.
Non Patent Literature 3
   Shiratori, I., et al., J. Exp. Med. 199 (2004) p.525-533
Non Patent Literature 4
   H.Arase, et al., Science, 296, 1323, (2002)

### Summary of Invention

However, acyclovir affects only virus-infected cells, and although acyclovir can terminate the virus-infected cells, it cannot prevent new infection with the virus. Further, recently, there has been reported a herpes virus resistant to acyclovir (Trends Microbiol. 1994 2: 481), requesting development of a new anti-herpes virus drug other than acyclovir.

Glycoprotein B (hereinafter abbreviated as "gB") and glycoprotein D (hereinafter abbreviated as "gD") are essential in infection with HSV-1. It is known that a receptor for gD is HVEM and nectin on a surface layer of a cell (R.I.Montgomery, et al., Cell, 87, 427, (1996), R.J.Geraghty, et al., Science, 280, 1618, (1998)). But a receptor that interacts with gB has not been known, although the existence of such receptor has been suggested (F.C.Bender, J.C., et al., J. Biol., 79, 11588, (2005)).

The present invention was made in view of the foregoing problems. An object of the present invention is to provide a herpes virus infection inhibitor, a method for inhibiting infection with herpes virus, and a representative use thereof.

In order to solve the foregoing problems, the inventors of the present invention has diligently studied and found that gB of herpes virus binds to PILR and PILR plays a significant role as an entry receptor when the herpes virus infects host cells. Further, the inventors have found that binding of gB with PILR on the host cells is important in herpes virus infection on the basis of the fact that herpes virus infection against peripheral blood mononuclear cells that express HVEM serving as a receptor for gD is inhibited by anti-PILR antibody. Thus, the inventors have completed the present invention. The present invention has been completed based on the above new finding and includes the following subject matters.

A herpes virus infection inhibitor of the present invention contains an effective component capable of binding to glycoprotein B or a receptor for glycoprotein B and capable of inhibiting interaction between glycoprotein B and the receptor for glycoprotein B.

gB is a component essential for infection with HSV-1. At the time of infection, virus binds to a receptor for gB included in host cells. The herpes virus infection inhibitor of the present invention contains the effective component capable of binding to glycoprotein B or a receptor for glycoprotein B. Accordingly, for example, in a case where the effective component is a substance bindable to gB, binding of the effective component to gB inhibits the interaction regardless of the kind of the receptor for gB.

Further, in a case where the effective component is a substance bindable to the receptor for gB for example, the receptor is blocked, which inhibits the interaction.

Therefore, regardless of whether the effective component is a later-mentioned anti-PILR antibody or not or whether the effective component is soluble PILR or not, it is possible to prevent infection of a host with herpes virus.

It is preferable to arrange the herpes virus infection inhibitor of the present invention so as to further include an effective component capable of binding to PILR that is a receptor for glycoprotein B and capable of inhibiting interaction between PILR and glycoprotein B.

With the arrangement, PILR is specifically blocked, and therefore binding of gB to PILR is inhibited, resulting in inhibition of the interaction between PILR and gB. Therefore, it is possible to very effectively prevent infection of a host with herpes virus.

Further, it is preferable to arrange the herpes virus infection inhibitor of the present invention so that the effective component is an anti-PILR antibody. With the arrangement, the anti-PILR antibody specifically binds to PILR through an antigen-antibody reaction. This inhibits biding of gB to PILR, resulting in inhibition of the interaction between PILR and gB. Therefore, it is possible to very effectively prevent infection of a host with herpes virus.

Further, it is preferable to arrange the herpes virus infection inhibitor of the present invention so as to further include an effective component capable of binding to glycoprotein B and capable of inhibiting interaction between a receptor for glycoprotein B and glycoprotein B.

Soluble PILR, anti-gB antibody etc. that is an effective component capable of binding to glycoprotein B and capable of inhibiting interaction between a receptor for glycoprotein B and glycoprotein B can bind to gB of herpes virus. Therefore, administering a herpes virus infection inhibitor containing these substances as effective components to a host allows inhibition of binding between PILR originally included in host cells and gB of herpes virus. Therefore, it is possible to prevent infection of a host with herpes virus.

Further, it is preferable to arrange the herpes virus infection inhibitor of the present invention so that the effective component is soluble PILR.

Since the soluble PILR can bind to gB of herpes virus, administering the herpes virus infection inhibitor containing these substances as effective components to a host allows inhibition of binding between PILR originally included in host cells and gB of herpes virus. Therefore, it is possible to prevent infection of a host with herpes virus.

Further, a method of the present invention for inhibiting infection with herpes virus includes the step of administering a herpes virus infection inhibitor of the present invention to a host and inhibiting interaction between a receptor for glycoprotein B and glycoprotein B in the host, so that infection of the host with herpes virus is inhibited.

With the arrangement, the herpes virus infection inhibitor binds to gB and/or the receptor for gB. Therefore, it is possible to inhibit interaction between gB of herpes virus and PILR. Therefore, it is possible to prevent infection of a host with herpes virus. In particular, it is possible to inhibit new infection of non-infected cells with herpes virus.

A receptor of the present invention for herpes virus is made of PILR and capable of binding to glycoprotein B contained in the herpes virus. Further, an animal or a cell of the present invention that is susceptible to herpes virus contains a receptor of the present invention for herpes virus.

The fact that PILR serves as a receptor for gB included in herpes virus when the herpes virus infects a host is finding found in the present invention for the first time. Therefore, the receptor can be preferably used in preparation etc. of herpes virus-susceptible cells for experimental use. Further, the animal or the cell susceptible to herpes virus can be preferably used in a test for inhibiting infection with herpes virus and other tests.

For a fuller understanding of the nature and advantages of the invention, reference should be made to the ensuing detailed description taken in conjunction with the accompanying drawings.

### Brief Description of Drawings

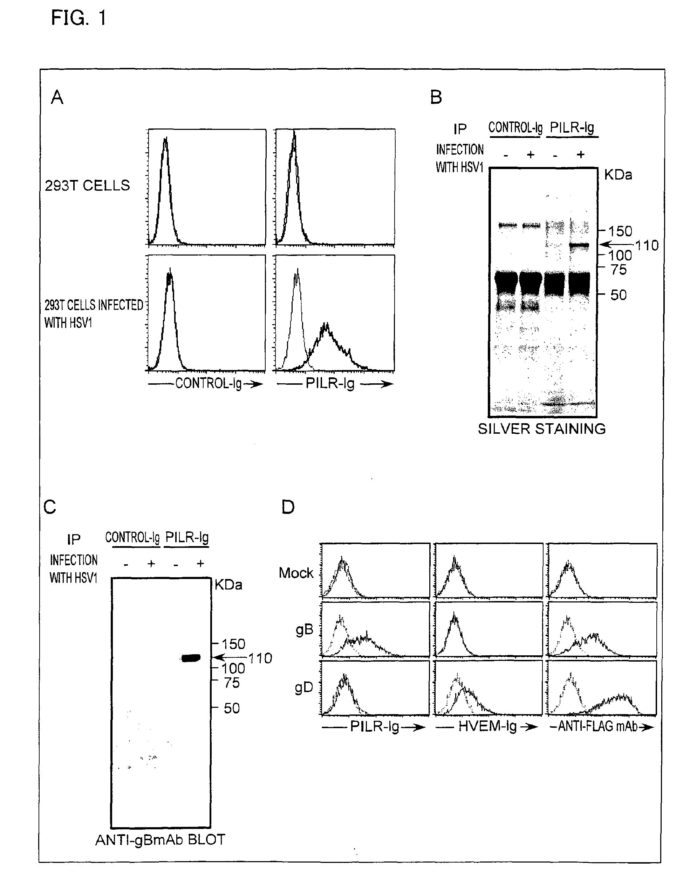

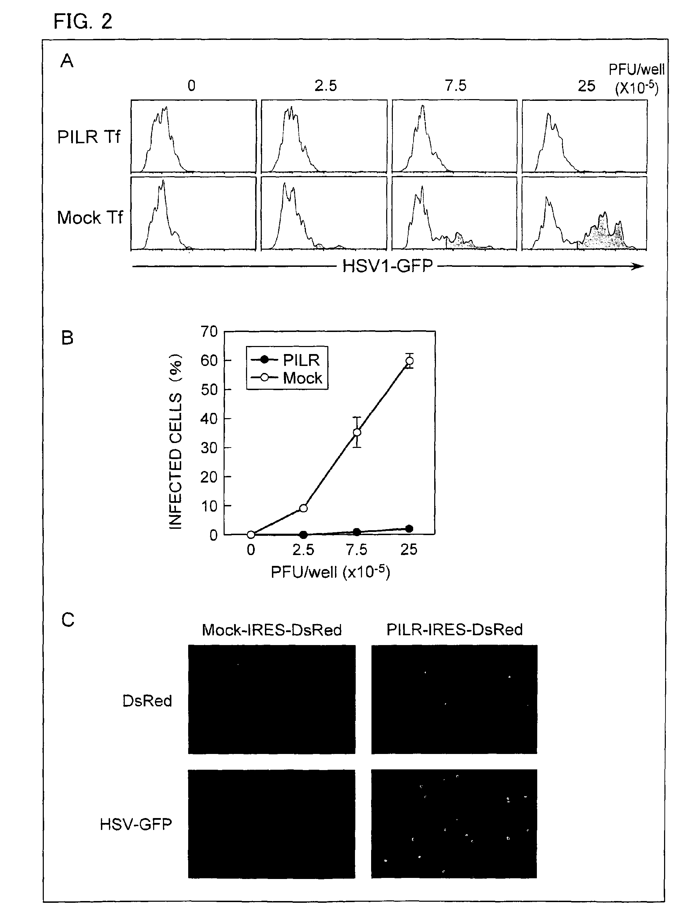

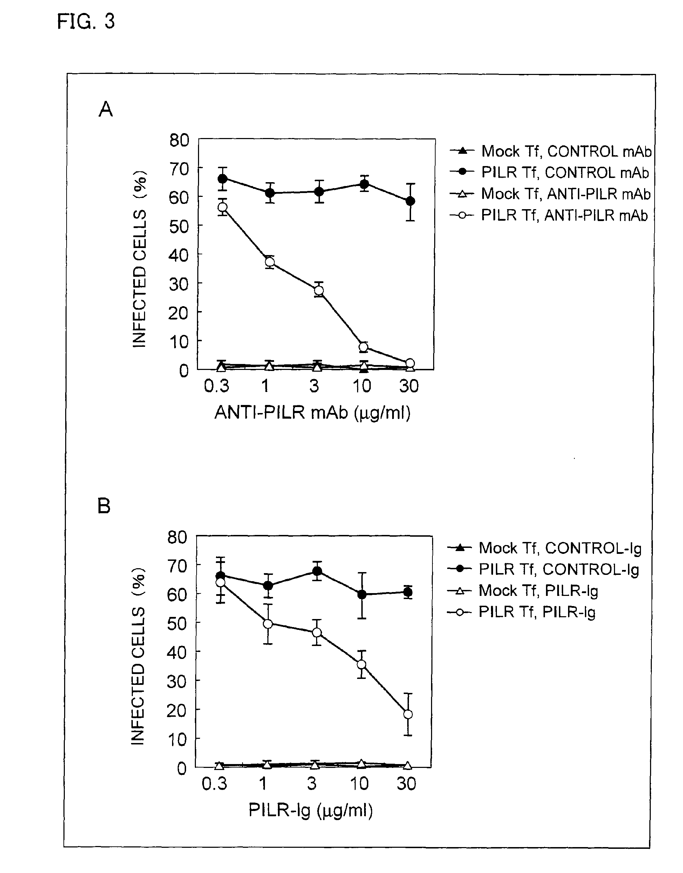

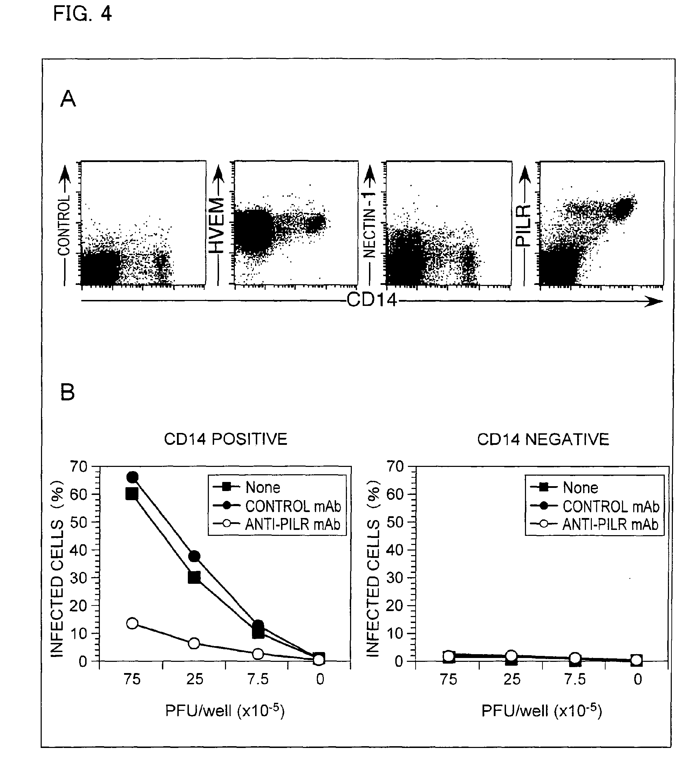

Fig. 1
   A of Fig. 1 shows that a ligand bindable to PILR is expressed in 293T cells infected with HSV-1. B of Fig. 1 shows that in a lysate of HSV-1-infected cells, not a control Ig (CD200-Ig) but PILR-Ig precipitated molecules of 110kDa, and the molecules were observed as a clear band in SDS-PAGE. C of Fig. 1 shows the result of westernblot analysis of a PILR-recognizing ligand included in HSV-1. D of Fig. 1 shows the result of analysis by anti-flag mAb of expressions of gB and gD on the surface of a cell.
Fig. 2
   A of Fig. 2 shows the result of analysis by flow cytometry, indicating that CHO-K1 cells to which human PILR had been transfected were effectively infected with HSV-1, but mock-transfected CHO-K1 cells were not infected with HSV-1. B of Fig. 2 shows a ratio of infected cells to CHO-K1 cells to which PILR had been transfected. C of Fig.2 shows the results of expression of DsRed and expression of GFP in CHO-K1 cells to which PILR-IRES-DsRed or Mock-IRES-DsRed had been transfected.
Fig. 3
   A of Fig. 3 shows the result of infecting CHO-K1 cells to which human PILR had been transfected with HSV-1 in the presence of anti-human PILR mAb (M4) or control mAb and examining to what degree the CHO-K1 cells were infected with HSV-1. B of Fig. 3 shows the result of inhibition by PILR-Ig of infection with HSV-1.
Fig. 4
   A of Fig. 4 shows that PILR was detected in CD+14 cells. B of Fig. 4 shows that infection of CD14+cells with HSV-1 was evidently blocked by anti-human PILR mAb.
Fig. 5

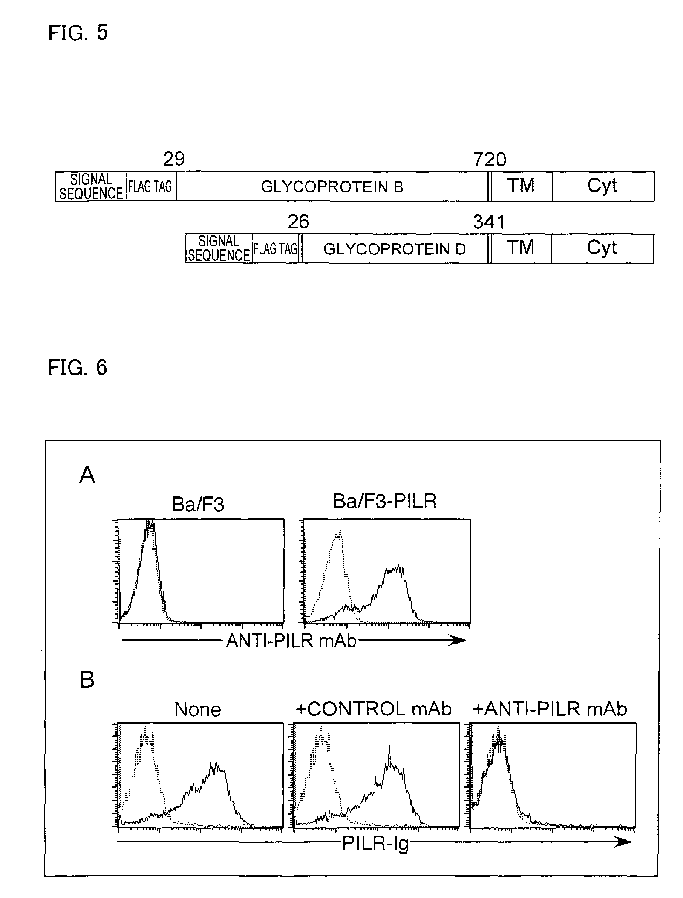

   Fig. 5 shows a contract used for expressions of extracellular regions of gB and gD.
Fig. 6
   A of Fig. 6 shows that Ba/F3 cells to which human PILR had been transfected were specifically recognized by anti-human PILR mAb (M4). B of Fig. 6 shows that anti-human PILR mAb (M4) blocked binding of PILR-Ig to cells to which gB had been transfected.
Fig. 7

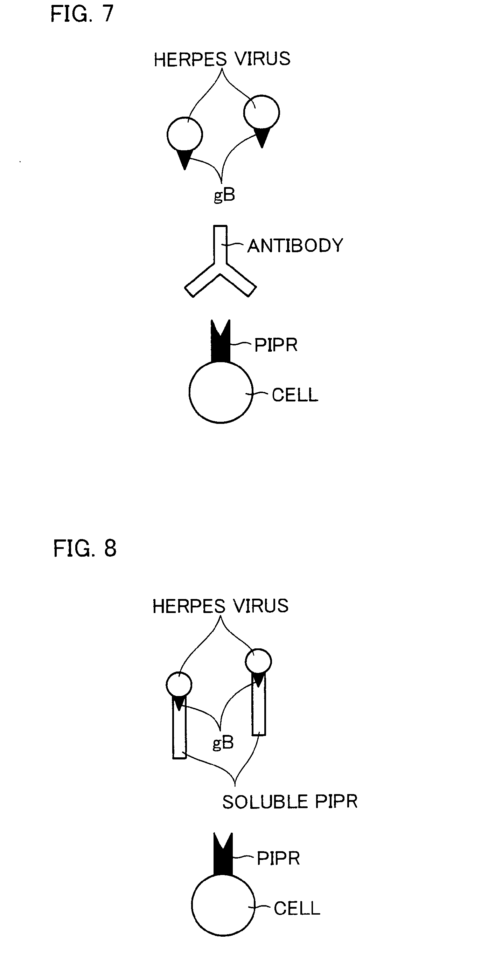

   Fig. 7 is a drawing schematically showing how an antigen-antibody reaction between anti-PILR antibody and PILR inhibits interaction between gB and PILR.
Fig. 8
   Fig. 8 is a drawing schematically showing how interaction between gB and PILR inhibits interaction between gB and PILR included in host cells.
Fig. 9

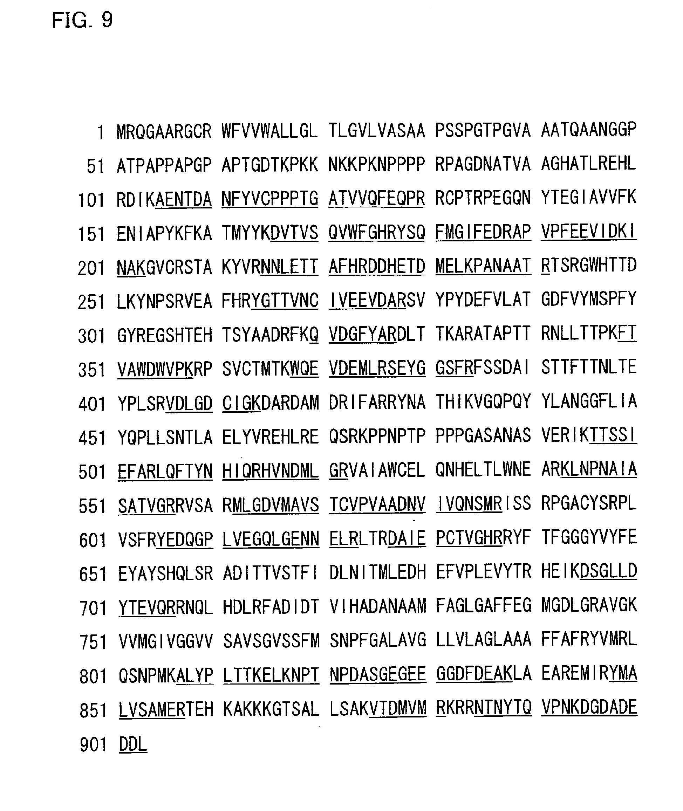

   Fig. 9 is a drawing showing an amino acid sequence of gB in HSV-1.
Fig. 10

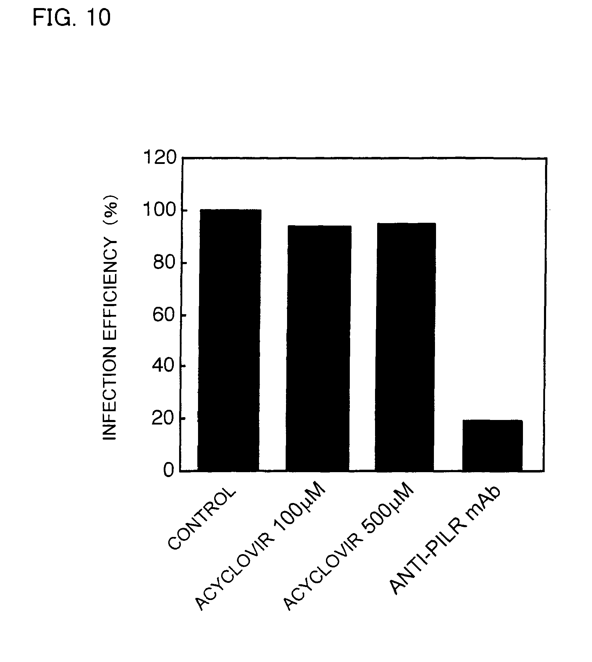

   Fig. 10 is a drawing showing that acyclovir terminates infected cells but cannot inhibit infection itself.

### Description of Embodiments

A herpes virus infection inhibitor of the present invention is an inhibitor containing an effective component capable of binding to glycoprotein B or a receptor for glycoprotein B and capable of inhibiting interaction between glycoprotein B and the receptor for glycoprotein B. The herpes virus infection inhibitor of the present invention includes a herpes virus infection inhibitor that contains as an effective component a substance for inhibiting interaction between PILR and glycoprotein B in herpes virus (examples of the substance include anti-PILR antibody, sugar chain having properties similar to those of anti-PILR antibody, other protein, other chemical substance etc.). Further, the herpes virus infection inhibitor of the present invention includes a herpes virus infection inhibitor that contains soluble PILR etc. as an effective component. The following initially explains the herpes virus infection inhibitor of the present invention.

### <Herpes virus infection inhibitor>

A herpes virus infection inhibitor of the present invention contains an effective component capable of binding to glycoprotein B or a receptor for glycoprotein B and capable of inhibiting interaction between glycoprotein B and the receptor for glycoprotein B.

Herpes virus is classified into three subfamilies: herpes simplex virus, cytomegalovirus, and lymphocryptovirus. Glycoprotein (gB) is a molecule common among the three subfamilies, and is a component essential for HSV-1 to infect a host. gB plays a significant role in infection with HSV-1 (R.I.Montgomery, et al., Cell, 87, 427, (1996), R.J.Geraghty, et al., Science, 280, 1618, (1998)).

The receptor for gB is not particularly limited. An example of the receptor is PILR. The receptor for gB other than PILR is a receptor immunoprecipitated by soluble gB from PILR-non-expressed cells to which the soluble gB can bind. The immunoprecipitated receptor can be easily identified by mass spectroscopy. Further, it is possible to identify the receptor by preparing a cDNA library from cells to which soluble gB bind and carrying out expression cloning.

Herein, the "soluble gB" is a protein obtained by fusing gB and IgG or gB from which an intracellular region and a transmembrane region are removed. The soluble gB can be prepared by a conventional and publicly known method, as in a case of later-mentioned soluble PILR.

For example, the soluble gB can be obtained by preparing expressed plasmid of chimera molecules of a gB extracellular region and an IgG-Fc region, introducing the expressed plasmid into Cos7 cells and culturing the plasmid, and then purifying the soluble gB from the resulting culture supernatant. Alternatively, the soluble gB can be obtained by preparing expressed plasmid of a gB extracellular region only, introducing the expressed plasmid into Cos7 cells and culturing the plasmid, and then purifying the soluble gB from the resulting culture supernatant.

In consideration of later-mentioned Examples, a receptor for the gB identified by the above method is expected to be an entry receptor for HSV-1 as in the case of PILR. Therefore, inhibition of interaction between a receptor for gB other than PILR and gB allows inhibiting infection of unknown receptor-expressed cells for gB with HSV.

The inventors of the present invention have uniquely found that gB of herpes virus binds to PILR and PILR plays an important role as an entry receptor when herpes virus infects host cells. Further, the inventors have found that binding of gB with PILR on the host cells is important in herpes virus infection on the basis of the fact that herpes virus infection against peripheral blood mononuclear cells that express HVEM serving as a receptor for gD is inhibited by an anti-PILR antibody. Therefore, the receptor is particularly preferably PILR.

The interaction between gB and the receptor for gB indicates binding between gB and the receptor for gB. The "binding" is a binding detected by a flow cytometry etc., in which a soluble receptor binds to gB or a binding detected by a flow cytometry etc., in which soluble gB binds to a receptor. "capable of inhibiting interaction" indicates capability to inhibit the binding. This indicates both a case where all gBs and all receptors for the gBs do not bind to each other, and a case where some of gBs and some of receptors for gBs do not bind to each other. That is, the number of the binding should be reduced in the case of using the infection inhibitor of the present invention compared with the case of using no infection inhibitor of the present invention.

The inhibition of the interaction can be confirmed by a conventional and publicly known method. An example of the method is a flow cytometry as explained in the later-mentioned Examples.

### (1-1. Herpes virus infection inhibitor containing an effective component capable of binding to a receptor for gB and capable of inhibiting interaction between gB and the receptor for gB)

In one embodiment of the present invention, the herpes virus infection inhibitor of the present invention contains an effective component capable of binding to a receptor for gB and capable of inhibiting interaction between gB and the receptor for gB. The effective component is not particularly limited. An example of the effective component is a component capable of binding to PILR and capable of inhibiting interaction between PILR and glycoprotein B.

Examples of such component include: an antibody bindable to the PILR and inhibits interaction between PILR and gB of herpes virus, i.e. an anti-PILR antibody; a sugar chain compound bindable to PILR; other protein; and other chemical substance.

In particular, since PILR has amino acids highly homologous to those of lectin such as Siglec, it is expected that PILR have a recognition mechanism similar to that of such lectin. Accordingly, it is expected that a sugar chain compound (e.g. derivatives of galactose and galactosamine) that is known as exhibiting competitive inhibition to lectin having homology with PILR, soluble protein of such lectin molecule etc. have the same function as that of the anti-PILR antibody as an inhibitor. The compound having the same function as that of the anti-PILR antibody can be chemically synthesized or produced as protein by a conventional and publicly known method.

The anti-PILR antibody is an antibody bindable to PILR and inhibits interaction between PILR and gB of herpes virus. Whether the anti-PILR antibody specifically binds to PILR or not can be confirmed by a flow cytometry using PILR-expressed cells as shown in A of Fig. 6.

As shown in the later-mentioned Examples, the inventors have found that PILR of host cells serves as a receptor for gB of herpes virus and plays an important role in infection of a host with herpes virus. Based on this finding, the inventors have found that use of an antigen-antibody reaction between the anti-PILR antibody and PILR allows inhibition of the interaction between gB and PILR, inhibiting infection with herpes virus.

Fig. 7 is a drawing schematically showing how the antigen-antibody reaction between the anti-PILR antibody and PILR inhibits the interaction between gB and PILR. Further, according to the later-mentioned Examples, it is considered that as with the anti-PILR antibody, a sugar chain and other protein and other chemical substance capable of binding to PILR can inhibit the interaction between gB and PILR. That is, any substance other than the anti-PILR antibody can inhibit infection with herpes virus as long as the substance is capable of binding to PILR as with the anti-PILR antibody.

Herpes virus is classified into three subfamilies: herpes simplex virus, cytomegalovirus, and lymphocryptovirus. gB is a molecule common among the three subfamilies. Therefore, herpes virus in the present specification may belong to any of the subfamilies. Among the subfamilies, herpes simplex virus is preferable. Herpes simplex virus may be HSV-1 or HSV-2.

In the present specification, "antibody" indicates immune globulin (IgA, IgD, IgE, IgG, IgM, and Fab fragments thereof, F(ab')₂ fragments thereof, Fc fragments thereof). Examples of the antibody include, but not limited to, a polyclonal antibody, a monoclonal antibody, a single chain antibody, an anti-idiotype antibody, and a humanized antibody.

A method for preparing the antibody may be any of various publicly known methods. For example, in the later-mentioned Examples, a mouse is immunized with PILR, a hybridoma is prepared from cells of immunized lymph nodes, clones of the hybridoma that recognizes PILR are gathered, a clone that inhibits binding of PILR-Ig to an extracellular region of gB is selected from the clones, and the selected clone is considered as an anti-PILR antibody.

As for a method for producing the antibody, it is preferable to purify the antibody by a publicly known method from a culture supernatant of clones of the hybridoma. Further, the antibody may be produced in such a manner that a polynucleotide encoding a polypeptide of the antibody is identified, the polynucleotide is introduced into host cells, and the polypeptide is expressed in the cells to produce the polypeptide.

The polypeptide may be produced in such a manner that a polynucleotide encoding a polypeptide of an antibody is identified from the antibody-producing clones and adopting its recombinant expressions. In a case of using the recombinant expressions, the method for producing polypeptide may include the step of introducing into host cells a vector into which a polynucleotide encoding a polypeptide of an antibody has been inserted.

The method for preparing a polynucleotide encoding a polypeptide of the antibody is not particularly limited. For example, a person skilled in the art can easily prepare a polynucleotide by identifying a polynucleotide encoding an antibody from PILR antibody-producing clones.

The polynucleotide may exist in the form of RNA (e.g. mRNA) or in the form of DNA (e.g. cDNA or genome DNA). DNA may be double strand or single strand. The single strand DNA or RNA may be a code strand (also known as sense strand) or a non-code strand (also known as antisense strand).

An example of the method for preparing a recombined expression vector includes, but not limited to, a method using plasmid, phage, or cosmid.

Specific kinds of the vector are not particularly limited, and a vector capable of expressing in host cells should be selected appropriately. That is, a promoter sequence should be selected appropriately in order to express a polynucleotide encoding a polypeptide of an antibody according to the kinds of host cells, and a vector in which the promoter sequence and the polynucleotide are put in various plasmids etc. should be used as an expression vector.

The expression vector may include at least one selection marker. Examples of the selection marker include: a dihydrofolate reductase gene or a neomycin-resistance gene in a case of culturing eucaryotic cells; and a tetracycline-resistance gene or an ampicillin-resistance gene in a case of culturing E. coli and other bacteria.

Use of the selection marker allows confirming whether the polynucleotide is introduced into host cells or not, and whether the polynucleotide is surely expressed in the host cells or not. The polypeptide may be expressed as fused polypeptide. For example, using green fluorescent polypeptide GFP (Green Fluorescent Protein) derived from Aequorea victoria or RFP (Red Fluorescent Protein) as a marker, the polypeptide may be expressed as GFP-fused polypeptide or RFP-fused polypeptide.

The host cells are not particularly limited and conventional and publicly known cells may be used appropriately. Specific examples of the host cells include, but not limited to, bacteria such as Escherichia coli; yeasts such as Saccharomyces cerevisiae and Schizosaccharomyces pombe; Caenorhabditis elegans; oocyte of Xenopus laevis; and cells derived from mammals. An appropriate culture medium for the host cells and appropriate conditions for culturing the host cells are well known in the field to which the present invention pertains.

A method for introducing the expression vector into host cells, i.e. a genetic transformation method is not particularly limited. A conventional and publicly known method such as an electroporation method, a calcium phosphate method, a liposome method, a DEAE dextran method, and retro virus method may be preferably used as the method.

It is preferable that the method for producing polypeptide further includes the step of purifying polypeptide from an extraction solution of cells or tissues containing the polypeptide. The step of purifying polypeptide preferably includes, but not limited to, the substeps of preparing a cell extraction liquid from cells and tissues by a well known method (e.g. breaking cells or tissues and then collecting a soluble fraction by centrifuge) and thereafter purifying polypeptide from the cell extraction liquid by a well known method (e.g. ammonium sulfate precipitation or ethanol precipitation, acid extraction, immunoprecipitation, negative ionic or positive ionic exchange chromatography, phosphocellulose chromatography, hydrophobic interaction chromatography, affinity chromatography, hydroxyapatite chromatography, and lectin chromatography). High performance liquid chromatography (HPLC) is preferably used for purification. The method for purifying polypeptide produced in the host cells varies according to what host is used and to the natures of polypeptide. It is possible to purify desired polypeptide relatively easily by use of tag etc.

Note that the method for producing the antibody (polypeptide) is not limited to the above method using the recombinant expressions, and may be a method producing the antibody by purifying the polypeptide from cells or tissues that express the antibody naturally. The method for purification may be any of the above techniques.

Other method for preparing the antibody is, for example, a method proposed by HarLow et al. in "Antibodies: A laboratory manual, Cold Spring Harbor Laboratory, New York (1988)" and a method proposed by Iwasaki et al. in "monoclonal antibody hybridoma and ELISA, Kodansha (1991)" etc.

A component other than the effective component is not particularly limited as long as it does not inhibit the effect yielded by the effective component. For example, the inhibitor may contain an emulsifier, an excipient etc. A method for mixing the effective component with other component is not particularly limited and may be a conventional and publicly known method. Further, the herpes virus infection inhibitor of the present invention has an action mechanism different from that of other anti-virus drug such as acyclovir and therefore can be used in combination with such other anti-virus drug.

### (1-2. Herpes virus infection inhibitor containing effective component capable of binding to gB and capable of inhibiting interaction between receptor for gB and gB)

In one embodiment of the present invention, the herpes virus infection inhibitor of the present invention contains an effective component capable of binding to gB and capable of inhibiting interaction between a receptor for gB and gB.

The effective component is not particularly limited as long as it is capable of binding to gB. Examples of the effective component include soluble PILR and anti-gB antibody.

As shown in the later-mentioned Examples, the inventors have found that PILR included in host cells serves as a receptor for gB of herpes virus, and plays an important role in infection of the host cells with herpes virus. Based on the finding, the inventors have found that supplying to a host soluble PILR that is a protein resulting from fusion of PILR and IgG and causing the soluble PILR to interact with gB of herpes virus in advance allows inhibiting interaction between gB and PILR included in the host cells, inhibiting infection with herpes virus. Fig. 8 is a drawing schematically showing how the interaction between gB and soluble PILR inhibits the interaction between gB and PILR included in the host cells.

In the later-mentioned Examples, the soluble PILR is used. However, the effective component is not limited to the soluble PILR, and any substance capable of binding to gB of herpes virus, such as an anti-gB antibody, can inhibit herpes virus from recognizing PILR, as the soluble PILR can inhibit.

It is easily expected from the later-mentioned Examples that use of the infection inhibitor containing the effective component allows a substance such as the anti-gB antibody for inhibiting interaction between gB of herpes virus and PILR to interact with gB of herpes virus, and gB is blocked by the substance such as the anti-gB antibody etc., inhibiting the interaction between PILR originally included in the host cells and gB of herpes virus. Therefore, in addition to the soluble PILR, any substance capable of binding to gB of herpes virus, such as the anti-gB antibody, can very effectively inhibit infection of a host with herpes virus.

The "soluble PILR" is a protein obtained by fusing PILR with IgG or PILR from which an intracellular region and a transmembrane region are removed. As shown in the later-mentioned Examples, the soluble PILR can be prepared by a conventional and publicly known method. For example, the soluble PILR may be prepared in such a manner that an expression plasmid of a chimera molecule between a PILR extracellular region and an IgG-Fc region is prepared, the expression plasmid is introduced into a Cos7 cell and cultured there, and then the soluble PILR is purified from the resulting culture supernatant. Alternatively, the soluble PILR may be prepared in such a manner that an expression plasmid including only extracellular region of PILR is prepared, introduced into a Cos7 cell and cultured there, and then the soluble PILR is purified from the resulting culture supernatant.

The amino acid sequence of human PILR is shown as SEQ ID No: 10. Amino acids from Q at the 20^{th} position to L at the 194^{th} position correspond to an extracellular region. Further, the amino acid sequence of human soluble PILR is shown as SEQ ID No: 11. Amino acids from M on the 1^{st} position to G on the 14^{th} position correspond to a signal sequence, amino acids from Q on the 27^{th} position to L on the 201^{st} position correspond to an extracellular region of human PILR, and amino acids from P on the 204^{th} position to K on the 434^{th} position correspond to human IgG1 Fc region.

The origin of PILR used for preparation of the soluble PILR is not particularly limited and may be human PILR, mouse PILR etc. PILR used for preparation of the soluble PILR may vary according to the kind of an organism whose infection with herpes virus is to be inhibited. The amino acid sequence of the human soluble PILR is shown by SEQ ID No: 11. Polypeptide made of an amino acid sequence with one or several amino acids replaced, deleted, inserted, and/or added in the SEQ ID No: 11 may also be used as the human soluble PILR. Soluble PILR derived from other organism can be prepared by fusing publicly known PILR with known IgG. Polypeptide made of an amino acid sequence with one or several amino acids replaced, deleted, inserted or added in the amino acid sequence in the prepared polypeptide (which may be hereinafter referred to as mutant) may also be used.

In the present specification, "with one or several amino acids replaced, deleted, inserted, or added" indicates that amino acid(s) in the number that allows replacement, deletion, insertion, or addition of an amino acid by a publicly known mutated polypeptide preparation method such as a region-specific mutagenesis method (the number is preferably 10 or less, more preferably 7 or less, and most preferably 5 or less) is replaced, deleted, inserted, or added.

It is well known in the field to which the present invention pertains that some amino acids in polypeptide can be easily modified without a significant influence on the structure or the function of the polypeptide. Further, it is well known in the field that, in addition to such artificial mutation, there is a mutant of a natural protein in which the structure or the function of the protein is not significantly changed.

A person skilled in the art can easily mutate one or more amino acids in an amino acid sequence of polypeptide by use of a well known technique. For example, with a publicly known point mutation method, it is possible to mutate any base of polynucleotide that encodes polypeptide. Further, it is possible to prepare a deletion mutant or an added mutant by designing a primer corresponding to any region of polynucleotide that encodes polypeptide. Further, with the method described in the present specification, it is possible to easily determine whether the prepared mutant has a desired activity or not.

A preferable mutant includes conservative or non-conservative replacement of amino acids, deletion, and/or addition. The mutation is preferably silent replacement, addition, and/or deletion, and more preferably conservative replacement.

The polypeptide may be obtained in such a manner that polynucleotide that encodes the polypeptide is introduced into host cells and the polypeptide is expressed in the cells. Alternatively, the polypeptide may be isolated and purified from cells, tissues etc. Further, the polypeptide may be chemically synthesized.

In other embodiment, the polypeptide may be recombinantly expressed in a modified form such as a fused protein. That is, the polypeptide may be polypeptide in which an amino acid sequence such as a tag is added to an end, especially to a side of an N-terminus of the polypeptide.

It is known that sugar chain modification plays an important role in expression of functions of a protein, especially a protein that expresses on the surface of a cell. The polypeptide includes sugar chain-modified polypeptide.

The method for producing the soluble PILR may be carried out by employing recombinant expressions using polynucleotide that encodes polypeptide of the soluble PILR or a mutant thereof. In a case of using the recombinant expressions, the method for producing polypeptide may include the step of introducing to host cells a vector to which polynucleotide that encodes polypeptide of the soluble PILR or a mutant thereof is inserted.

The method for producing polynucleotide that encodes polypeptide of the soluble PILR or a mutant thereof is not particularly limited. For example, a person skilled in the art can easily prepare polynucleotide that encodes polypeptide of the soluble PILR or a mutant thereof on a basis of descriptions of conventional and publicly known soluble PILR. The form of polynucleotide, the kind of a vector, host cells, a genetic transformation method, purification of polypeptide etc. are the same as those explained in (1-1).

The gB antibody may be prepared by any of various publicly known methods. For example, the anti-gB antibody may be prepared in such a manner that a mouse is immunized with gB, a hybridoma is prepared from a cell of an immunized lymph node, and clones of the hybridoma that recognizes gB are gathered.

A component other than the effective component is not particularly limited as long as it does not inhibit the effect yielded by the effective component. For example, the inhibitor may contain an emulsifier, an excipient etc. A method for mixing the effect component with other component is not particularly limited and may be a conventional and publicly known method. Further, the herpes virus infection inhibitor of the present invention has an action mechanism different from that of other anti-virus drug such as acyclovir and therefore can be used in combination with such other anti-virus drug.

### (1-3. Herpes virus infection inhibitor containing polynucleotide that encodes polypeptide)

A drug containing polynucleotide that encodes polypeptide that is the above-explained effective component serves as a herpes virus infection inhibitor. The herpes virus infection inhibitor containing polynucleotide that encodes polypeptide that is the effective component can produce effective components such as an anti-PILR antibody, an anti-gB antibody, and soluble PILR, when the herpes virus infection inhibitor is introduced into host cells by use of recombinant expressions and expressed therein, as explained in (1-1) or (1-2). Therefore, such herpes virus infection inhibitor can inhibit infection with herpes virus, as with the herpes virus infection inhibitor explained in (1-1) or (1-2). For example, the anti-PILR antibody is produced from a culture supernatant of an anti-PILR antibody-producing hybridoma and an anti-PILR antibody-producing cell. The polynucleotide can be easily prepared by a person skilled in the art based on the amino acid sequence of polypeptide that is the effective component.

### <2. Receptor for herpes virus, animal or cell susceptible to herpes virus>

### (2-1. Receptor for herpes virus)

A receptor of the present invention for herpes virus consists of PILR, and is capable of binding to glycoprotein B included in herpes virus. As shown in the later-mentioned Examples, the fact that PILR serves as a receptor for herpes virus infecting host cells is found in the present invention for the first time.

The receptor can be preferably used for preparation etc. of herpes virus-susceptible cells for testing.

PILR may be obtained in such a manner that polynucleotide encoding PILR is introduced into host cells and its polypeptide is expressed in the cells, or may be obtained by isolating and purifying PILR from cells, tissues etc. For example, PILR cloned in a human and a mouse (Non-patent Literatures 1 and 2) etc. may be used. The PILR may be chemically synthesized.

A method for producing PILR is not particularly limited. For example, recombinant expressions that use polynucleotide encoding polypeptide of PILR or a mutant thereof may be used. In the case of using the recombinant expressions, the method for producing polypeptide may include the step of introducing to host cells a vector to which polynucleotide encoding polypeptide of PILR or a mutant thereof is inserted.

### (2-2. Animal or cell susceptible to herpes virus)

As explained in (1-1), the fact that PILR serves as a receptor for herpes virus infecting host cells has been found in the present invention for the first time. Therefore, a cell or an animal in which polynucleotide encoding PILR is introduced into a host cell and its polypeptide is expressed in the cell has a feature of being susceptible to herpes virus. Accordingly, the animal or the cell susceptible to herpes virus can be preferably used for a test of inhibiting infection with herpes virus and other tests.

The kinds of the animal are not particularly limited. Examples of the animal include dogs, chickens, monkeys, mice, and rats that are generally used as test animals. The cell is not particularly limited as long as PILR is expressed in the cell.

The method for obtaining the animal or the cell is not particularly limited. For example, the recombinant expression vector may be introduced into a host, transformed and expressed therein.

### <3. Method for inhibiting infection with herpes virus>

In the method of the present invention for inhibiting herpes virus infection, the herpes virus infection inhibitor of the present invention is supplied to a host, and interaction between a receptor for gB and gB is inhibited in the host so as to inhibit infection of the host with herpes virus.

In one embodiment of the present invention, the method of the present invention for inhibiting infection with herpes virus is a method for inhibiting infection of a host with herpes virus by use of an antigen-antibody reaction between PILR and an anti-PILR antibody. As described above, the present invention has for the first time clarified the fact that PILR serves as a receptor for gB of herpes virus. With the method, PILR is blocked by the anti-PILR antibody, and therefore the interaction between PILR of a host cell and gB of herpes virus is inhibited. This allows very effectively inhibiting infection of a host with herpes virus.

An example of the anti-PILR-antibody is an antibody explained in (1-1) for example. The antigen-antibody reaction can be caused by mixing the anti-PILR antibody with the host cell. Whether infection with herpes virus is inhibited in the host cell having been subjected to the antigen-antibody reaction can be examined by conventional and publicly known means such as a flow cytometry.

In one embodiment of the present invention, the method of the present invention for inhibiting infection with herpes virus is a method for inhibiting infection of a host with herpes virus by use of interaction between soluble PILR and gB of herpes virus. With the method, the interaction between the soluble PILR and gB of herpes virus makes the soluble PILR block gB, inhibiting interaction between PILR originally included in a host cell and gB. This allows very effectively inhibiting infection of the host with herpes virus.

The soluble PILR may be the soluble PILR described in (1-2) for example. Whether the soluble PILR inhibits infection with herpes virus or not can be confirmed by a conventional and publicly known method such as immunoprecipitation.

The effective component of the infection inhibitor used in the method of the present invention for inhibiting infection with herpes virus is not limited to the anti-PILR that inhibits the interaction between gB of herpes virus and PILR and to the soluble PILR. It is easily expected from the later-mentioned Examples that the method of the present invention for inhibiting infection with herpes virus allows any substance that inhibits the interaction between gB of herpes virus and PILR to inhibit infection with herpes virus. As with the anti-PILR antibody and the soluble PILR, such substance can inhibit the interaction between PILR originally included in a host cell and gB. This allows very effectively inhibit infection of the host with herpes virus.

Examples of such substance include the anti-gB antibody that inhibits the interaction between gB of herpes virus and PILR, the sugar chain compound, and the chemical substance that are described in (1-1) and (1-2). Further, the effective component may be polynucleotide that encodes polypeptide that is an effective component as described in (1-3).

How to administer the infection inhibitor to a host is not particularly limited. For example, the infection inhibitor may be administered to the host by application, injection, oral ingestion etc.

It is preferable that the infection inhibitor is purified by the conventional and publicly known method described in (1-1) and the purified infection inhibitor is administered as a drug to a host. For example, it is general that a purified antibody or a purified soluble PILR is administered as a drug to the host. Examples of the host are not particularly limited as long as the host is an animal and may include dogs, chickens, monkeys, mice, rats, and humans. Mammals are preferably preferable.

### <4. Use of the present invention>

Acyclovir is most commonly used as a therapeutic drug for herpes virus. Acyclovir inhibits duplication of DNA, but is effective to only cells infected with herpes virus, and cannot inhibit new cells from being infected with herpes virus. That is, although acyclovir terminates cells infected with herpes virus, virus coming from the terminated cells still has an ability to infect other cells. Therefore, a new infection inhibitor and a new method for inhibiting infection that are described in the present invention are important. Further, recently, herpes virus resistive to acyclovir has been reported. This requires an infection inhibitor having an action mechanism different from that of acyclovir.

It was found by the present invention that when herpes virus infects a host cell, gB of virus binds to PILR of the host cell. The herpes virus infection inhibitor of the present invention contains as an effective component a substance that is capable of binding to gB or a receptor for gB and is capable of inhibiting interaction between gB and the receptor for gB. Examples of the effective component include an anti-PILR antibody, a sugar chain having properties similar to those of the anti-PILR antibody, other protein having properties similar to those of the anti-PILR antibody, chemical substance having properties similar to those of the anti-PILR antibody, soluble PILR, and an anti-gB antibody.

The anti-PILR antibody, the sugar chain having properties similar to those of the anti-PILR antibody, other protein having properties similar to those of the anti-PILR antibody, and the chemical substance having properties similar to those of the anti-PILR antibody specifically recognize PILR included in a host cell. The soluble PILR and the anti-gB antibody specifically recognize gB of herpes virus. Consequently, the infection inhibitor of the present invention can inhibit new infection with herpes virus. Therefore, the present invention is useful for treatment of various herpes virus infection diseases.

In a case where the herpes virus infection inhibitor of the present invention contains the anti-PILR antibody as an effective component, the content of the anti-PILR antibody is not particularly limited, but preferably not less than 100mg and not more than 1000mg per one usage. Further, in a case where the herpes virus infection inhibitor of the present invention contains the soluble PILR as an effective component, the content of the soluble PILR is not particularly limited, but preferably not less than 100mg and not more than 5000mg per one usage.

Note that it is preferable not to use the anti-PILR antibody and the soluble PILR in combination in the herpes virus infection inhibitor of the present invention since the anti-PILR antibody and the soluble PILR oppose each other. A component other than the effective component is not particularly limited as long as the component does not inhibit the effect of the effective component. A method for mixing the effective component with other component is not particularly limited and may be a conventional and publicly known method. Further, since the herpes virus infection inhibitor of the present invention has an action mechanism different from that of other anti-virus drug such as acyclovir, the herpes virus infection inhibitor of the present invention may be used in combination with other anti-virus drug.

The following explains Examples in order to further detail the Embodiments of the present invention. The present invention is not limited to the following Examples and may be modified variously in its details. Further, the present invention is not limited to the above embodiments and various modifications are possible within the scope of the claims. An embodiment based on a proper combination of technical means disclosed in different embodiments is encompassed in the technical scope of the present invention.

### [Examples]

The following further details the present invention with reference to Examples. The present invention is not limited to the following Examples. Operation that is not particularly mentioned in the following explanation was carried out according to a conventional and publicly known technique. Reagents and devices were used according to manuals attached thereto. Cells used in the following Examples were cultured in an RPMI1640 medium containing 10% of FCS except where otherwise specified.

### [Virus]

The following Examples used wild-type HSV-1(F) and HSV-1(F) including GFP (Green Fluorescent Protein) as a marker (M. Tanaka, et al., Microbes. Infect. 6, 485, 2004). Potency of virus was determined by a conventional and publicly known method with use of Vero cells. HSV-1(F) including GFP (Green Fluorescent Protein) as a marker is hereinafter referred to as "HSV-1-GFP".

### [Plasmid]

pMXs-mouse PILR-IRES-GFP and pME18S-mouse PILR-IgG were prepared by a method described in I.Shiratori et al., J.Exp. Med. 199, 525,2004. cDNA fragment of human PILR was obtained by PCR amplification of cDNA of human PBMC (peripheral blood mononuclear cells) with use of a sense primer made of a sequence (5'-AAT GAA TTC AAC AAG GCC ATG GGT CGG-3') described in SEQ ID No: 2 and an anti-sense primer made of a sequence (5'-AAT AAT GCG GCC GCA GGG CTG TCC ATT GGT TAG G-3') described in SEQ ID No: 3. The obtained cDNA fragment of human PILR was inserted between EcoRI sites and NotI sites of pMXs-IRES-GFP and pMXs-IRES-DsRed, and pMXs-human PILR-IRES-GFP and pMXs-human PILR-IRES-DsRed were prepared.

cDNA fragment corresponding to an extracellular region of human PILR was obtained by PCR amplification of pMXs-human PILR-IRES-GFP with use of a sense primer made of a sequence (5'- AAT CTC GAG CAG CCT AGT GGC TCC ACA GG-3') described in SEQ ID No: 4 and an anti-sense primer made of a sequence (5'- AAT AAT CTC GAG AAC CCT GAT GGC AGT GTC-3') described in SEQ ID No:: 5.

The fragment was inserted into an XhoI cloning site of a modified pME18S expression vector that contains a mouse C150 reader segment at N-terminus and contains a human IgG1-Fc segment at C-terminus, and thus pME18S-human PILR-IgG was prepared.

cDNA fragments corresponding to extracellular regions of glycoprotein B and glycoprotein D of HSV-1 was obtained by PCR amplification with use of the following primers. (1) Primer used for amplification of glycoprotein B: a sense primer made of a sequence (5'- AAT CTC GAG GCG GCT CCG AGT TCC CCC GGC A-3') described in SEQ ID No: 6 and an anti-sense primer made of a sequence (5'- AAT GAA TTC CGT GTC GAT GTC GGC GAA GC-3') described in SEQ ID No: 7. (2) Primer used for amplification of glycoprotein D: a sense primer made of a sequence (5'- AAT CTC GAG AAA TAT GCC TTG GTG GAT GC-3') described in SEQ ID No: 8 and an anti-sense primer made of a sequence (5'- AAT CTC GAG CAT GTT GTT CGG GGT GGC CG-3') described in SEQ ID No: 9.

The cDNA fragment was inserted into an XhoI or XhoI-EcoRV cloning site of a modified pME18S expression vector having a BM40 reader segment and a FLAG-tag sequence at an N-terminus and having transmembrane and an intracellular segment of mouse PILR.

### [Cell line]

293T cells and COS-7 were purchased from RIKEN Cell Bank. CHO-K1 (JCRB No. JCRB9018) was purchased from Health Science Research Resources Bank. CHO-K1 cells without ligands for PILR were obtained by a cell sorter (FACSAria). The CHO-K1 cells were stained with PILR-Ig and PE-labeled anti-human IgG antibody, and then non-stained cells were collected by the cell sorter. PBMC (peripheral blood mononuclear cells) were obtained by centrifuge with use of Ficoll-Perque PLUS (manufactured by Amersham Biosciences) according to the attached manual.

### [Soluble PILR]

pME18S-human PILR-IgG or pME18S-mouse PILR-IgG was transiently transfected into COS-7 cells. 2 days later, culture supernatant was collected and the collected supernatant was regarded as soluble human PILR (hereinafter referred to as "human PILR-Ig") or soluble mouse PILR (hereinafter referred to as "mouse PILR-Ig") and used in the following operation. Human PILR-Ig and mouse PILR-Ig were purified by a protein A column. Purified human CD200-Ig fusion protein (I. Shiratori et al., J. Immunol. 175, 4441, 2005) was used as a control.

### [Antibody]

A BALB/C mouse was immunized with human PILR-Ig using TiterMax Gold as an adjuvant. 2 weeks later, lymph node cells were fused with SP2/0 to obtain a clone to bind to Ba/ F3 cells to which human PILR was transfected. Out of clones that bind to human PILR, a specific clone that blocked binding of PILR-Ig to cells expressing extracellular regions of gB was selected. In the present Example, the clone was M4 that was IgG1 clone. Anti-FlagM2 antibody (mouse IgG1, produced by Sigma) was used as a control monoclonal antibody (hereinafter abbreviated as "mAb").

### [Transfection and infection]

CHO-K1 cells were transiently transfected in a 10% FCS-containing F-12 culture medium using Lipofectamine 2000 (produced by Invitrogen) or GeneJuice (produced by Novagen). 1 day after the transfection, the 10% FCS-containing F-12 culture medium was replaced with a 1% FCS-containing F-12 culture medium. 2 days after the transfection, the transfectant was mixed with HSV-GFP and centrifuged at 32°C at 2500rpm for 2 hours. Thus, the transfectant was infected with HSV-GFP.

PBMC (peripheral blood mononuclear cells) were obtained by centrifuge with Ficoll-Perque PLUS (manufactured by Amersham Biosciences) according to a method described in the manual. CD14+cells and CD14-cells were separated by use of a MACS purification system (manufactured by Miltenyi Biotec). The purified PBMC was cultured in a 1% FCS-containing advanced RPMI1640 culture medium for 2 hours and was mixed with HSV-GFP and centrifuged at 32°C at 2500rpm for 2 hours and thus infected with HSV-GFP. 12 hours after the infection, the cells were fixed with paraformaldehyde, and expression of GFP or DsRed was analyzed by use of a FACSCalibur (manufactured by Becton Dickinson).

### [Flow cytometry]

The cells were cultured with PILR-human Ig Fc fused protein or a primary mouse monoclonal antibody, and then cultured with PE-labeled anti-human IgG antibody or a PE-labeled anti-mouse IgG antibody. The stained cells were analyzed by use of a FACSCalibur (manufactured by Becton Dickinson).

### [Immunoprecipitation and immunoplotting]

The cells were dissolved in a solution buffer (20mM tris, 150mM sodium chloride, pH7.5) containing 1% Briji98 (produced by Sigma). The dissolved cells were immunoprecipitated with a mouse PILR-Ig. The precipitum obtained by the immunoprecipitation was eluted by boiling the precipitum together with an SDS-PAGE sample buffer, and was separated on 10% polyacrylic amide gel. The gel was directly silver-stained (stain solution was produced by Bio-Rad), transferred onto a PVDF film (produced by Millipore), and the PVDF film was blotted with an anti-gBmAb (Clone 1105, Rumbauhg-Goodwin Institute).

### [Digestion in gel and mass spectrometry]

A sample for mass spectrometry was prepared by trypsin digestion through the method described in Matsumoto M et al. Proteomics. 2005 5: 4145, and the mass of the sample was analyzed by nano-LC (Ultimate, LC packing) and ESI-Q-Tof MS/MS (Q-Tof Ultima API, Micromass). The mass of peptide and amino acid sequence were analyzed by use of a MASCOT program (Matrix Science Ltd).

### [Example 1: Binding of glycoprotein B to PILR]

First, a flow cytometric analysis was carried out so as to determine whether an HSV-1-infected 293T cell binds to PILR-Ig.

A of Fig. 1 shows that an HSV-1-infected 293T cell expresses a ligand bindable to PILR. This indicates that the HSV-1 has the ligand bindable to the PILR, or that the HSV-1 induces a cell to express the ligand bindable to the PILR.

Next, an analysis for identification of the ligand bindable to the PILR, which ligand was expressed in the HSV-1-infected cell, was carried out. Each lysate of the HSV-1-infected cell or a non-infected 293T cell was lysed with a surfactant, and then immunoprecipitated together with PILR-Ig. Then, each immunoprecipitate thus obtained was subjected to SDS-PAGE, and stained with silver so as to be analyzed.

B of Fig. 1 shows that, in the lysate of the HSV-1-infected cell, the PILR-Ig caused precipitation of a molecule (protein) of 110kDa, not a control Ig (CD200-Ig), and that the protein was clearly found as a band in the SDS-PAGE.

The protein of 110kDa was subjected to an amino acid analysis by an LC/MS/MS mass spectrometry so that its amino acid sequence can be analyzed. Fig. 9 and SEQ ID No: 1 show an amino acid sequence of gB of HSV-1. In Fig. 9, underlined amino acids are those identified in the amino acid analysis. High homology between the underlined amino acids and the sequences shown in SEQ ID No:1 show that the band is gB of HSV-1.

Molecules of 110kDa were blotted with anti-gBmAB in order to confirm that the protein of 110kDa is gB. C of Fig. 1 shows the result of western blot analysis on PILR-recognizing ligand included in HSV-1. The PILR-recognizing ligand expressed on the HSV-1 infected cell was immunoprecipitated with PILR-Ig or control Ig (CD200-Ig). The immunoprecipitate was analyzed with SDS-PAGE, and blotted with the anti-gBmAB. As is seen from C of Fig. 1, the protein of 110kDa was evidently blotted with the anti-gBmAB.

Subsequently, analysis was carried out so as to determine whether gB expressed on the surface of a cell was recognized specifically by PILR-Ig or not. When gB was transfected to a 293T cell, the entire length of gB was not expressed on the surface of the cell. Therefore, there was prepared a construct expressing a gB extracellular region with a Flag tag coupled with a transmembrane region and a cytoplasmic region of PILR, and the construct was transfected to the 293 cell.

Similarly, gD which is known to play an important role in infection with HSV-1 by binding to HVEM and nectin on the surface of a cell, was expressed on the 293 cell by using a construct in which a extracellular region was constructed as in the case of gB.

Fig. 5 is a construct used to express extracellular regions of gB and gD. A signal sequence and a FLAG tag (DYKDDDDK) were added to an N-terminus of the extracellular region (29^{th}- to 720^{th}-amino acids) of gB and to an N-terminus of the extracellular region (26^{th}- to 341^{st}-amino acids) of gD. The 293T cell to which the FLAG-tagged extracellular regions of gB and gD were transfected was stained with PILR-Ig, HVEM-Ig, and anti-FLAG mAb (Clone M2, Sigma) and analyzed by flow cytometry.

D of Fig. 1 shows the result of the analysis. Right column of D of Fig. 1 shows the result of analyzing that both of gB and gD are expressed on the surface of a cell with use of anti-FLAG mAb. Left column of D of Fig. 1 shows that PILR-Ig bound to a cell expressing gB instead of gD, indicating that PILR specifically bound to gB. On the other hand, central column of D of Fig. 1 shows that HVEM-Ig bound to a cell expressing gD instead of gB. Accordingly, data shown in D of Fig. 1 shows that PILR binds to gB.

gB and gD are essential when HSV-1 infects a host, and gB plays an important role in infection with HSV-1 (R.I.Montgomery, et al., Cell, 87, 427, (1996), R.J.Geraghty, et al., Science, 280, 1618, (1998)). However, although the existence of specific molecules that interact with gB had been suggested (F.C.Bender, J.C., et al., J. Biol., 79, 11588, (2005)), such specific molecules had not been known. The fact that specific molecules that interact with gB are PILR was found by the present invention for the first time.

### [Example 2: Confirmation of role of PILR in HSV-1 infection]

Since some CHO-K1 cells express an unknown ligand bindable to PILR, the inventors of the present invention have purified by a cell sorter CHO-K1 cells without a ligand bindable to PILR, and transfected PILR to the CHO-K1 cells without a ligand bindable to PILR in order to avoid interaction between PILR and a ligand existing on the surface of a CHO-K1 cell.

The CHO-K1 cells without a ligand bindable to PILR were transiently transfected using pMx-IRES-DsRed expression vector including human PILR. The transfected CHO-K1 cells were transfected using HSV-1-GFP, and cells expressing GFP in DsRed positive cells were analyzed by flow cytometry. A of Fig. 2 shows the result of the analysis by flow cytometry, indicating that the CHO-k1 cells to which human PILR had been transfected got infected with HSV-1 effectively, but mock-transfected CHO-K1 cells did not get infected with HSV-1.

B of Fig. 2 shows a ratio of infected cells in the CHO-K1 cells to which PILR had been transfected.

Subsequently, the PILR-IRES-DsRed or the Mock-IRES-DsRed was transfected to the CHO-K1 cells, and cells expressing DsRed were purified by a cell sorter. The transfected CHO-K1 cells were caused to be infected with HSV-1-GFP, and expression of GFP was analyzed by a fluorescent microscope.

C of Fig. 2 shows the result of expression of DsRed and expression of GFP in the CHO-K1 cells to which the PILR-IRES-DsRed or the Mock-IRES-DsRed was transfected. As is seen from C of Fig. 2, the CHO-K1 cells to which the PILR-IRES-DsRed had been transfected got infected with HSV-1-GFP, but the CHO-K1 cells to which the Mock-IRES-DsRed had been transfected did not got infected with HSV-1-GFP.

The above result clearly showed that cells expressing PILR were sufficiently infected with HSV-1. Accordingly, analysis was carried out to determine whether infection with HSV-1 can be blocked by anti-PILRmAb or PILR-Ig fused protein.

In order to carry out the analysis, anti-PILRmAb that specifically blocks interaction between human PILR and gB was established. A of Fig. 6 shows that Ba/F3 cells to which human PILR had been transfected were recognized specifically by anti-human PILRmAb (M4). Ba/F3 cells to which PILR had been transfected were mixed with anti-human PILRmAb (M4), and then PE-labeled anti-mouse IgG antibody was added to the mixture, which was stained and analyzed by flow cytometry. M4 antibody did not bind to Ba/F3 parent strain, but specifically recognized the Ba/F3 cells to which PILR had been transfected.

B of Fig. 6 shows that the anti-human PILR mAb (M4) blocked binding of PILR-Ig to cells to which gB had been transfected. PILR-Ig was cultured together with the anti-human PILRmAb (M4), and then mixed with Ba/F3 cells to which an extracellular region of gB had been transfected. Thereafter, PE-labeled anti-human IgG antibody was added to the mixture, which was stained and analyzed by flow cytometry. M4 antibody completely inhibited binding of PILR-Ig to the Ba/F3 cells to which gB had been transfected.

A of Fig. 3 shows the result of examination on the degree of infection when the CHO-K1 cells to which human PILR had been transfected were infected with HSV-1 in the presence of the anti-human PILR mAb (M4) or control mAb. Human PILR was transiently transfected to the CHO-K1 cells by using pMx-IRES-DsRed expression vector, and the CHO-K1 cells were caused to be infected with HSV-1 by using HSV-1-GFP in the presence of anti-human PILR mAb with various concentrations or control mAb. The ratio of HSV-1-infected cells was analyzed by flow cytometry. As shown in A of Fig. 3, infection with HSV-1 was completely blocked by anti-human PILR mAb in a concentration-dependent manner.

The similar result was observed in a case where the CHO-K1 cells to which PILR had been transfected were caused to be infected with HSV-1 and the infected cells were observed by a fluorescent microscope. B of Fig. 3 shows the result of inhibition by PILR-Ig of infection with HSV-1. The CHO-K1 cells to which PILR had been transfected were caused to be infected with HSV-1 in the presence of PILR-Ig with various concentrations or Ig-fused protein without PILR that was a control. The ratio of the infected cells was analyzed by flow cytometry. Since PILR-Ig directly binds to gB of HSV-1, it is considered that there is a possibility that PILR-Ig blocks interaction between HSV-1 and PILR on the surface of a cell.

As is seen from B of Fig. 3, infection of the PILR-transfected CHO-K1 cells with HSV-1 was not blocked by the control Ig-fused protein, but was significantly blocked by the PILR-Ig-fused protein in a concentration-dependent manner. This indicates that the interaction between PILR and gB has a relationship with infection with HSV-1.

Subsequently, whether PILR is related to infection of cells expressing endogenous PILR was examined.

It is reported that PILR is mainly expressed in mononuclear cells, granulocytes, and dendritic cells (Non-patent Literature 2). Accordingly, in order to examine expression of PILR, human peripheral blood mononuclear cells (PBMC) were stained with anti-CD 14 antibody and anti-PILR antibody. Further, in order to examine expression of HVEM and Nectin-1, PBMC was stained with anti-CD14 antibody and anti-HVEM antibody or anti-Nectin antibody. As shown in A of Fig. 4, PILR was detected in CD14+cells. Further, it was confirmed that CD14+cells expressed HVEM and weakly expressed Nectin-1.

Subsequently, analysis was made so as to determine whether PILR is related to infection of mononuclear cells expressing both HVEM and PILR with HSV-1. That is, newly separated CD14+cells and CD14-cells were caused to be infected with HSV-1-GFP both in a case where anti-human PILR mAb or control mAb (each 10µg/ml) exists and a case where the anti-human PILR mAb or the control mAb does not exist, and a ratio of infected cells was analyzed by flow cytometry.

As shown in B of Fig. 4, infection of CD14+cells with HSV-1 was evidently blocked by anti-human PILR mAb. On the other hand, a ratio of infection of CD14-cells mainly made of lymphocytes with HSV-1 was low, and anti-human PILR mAb showed almost no effect with respect to the infection with HSV-1. These data suggested that expression of HVEM only, that had been known as a receptor for gD and had been reported as relating to infection with HSV-1, is not sufficient for infection with HSV-1, and that binding between gB and a cell ligand plays an important role in infection with HSV-1.

### [Example 3: inhibition by anti-PILRα antibody of infection with HSV-1]

In the present Example, acyclovir was used as a control in a test in which infection with HSV-1 was inhibited by anti-PILR antibody. The test confirmed that although acyclovir terminates HSV-1-infected cells, acyclovir cannot inhibit infection itself.

HSV-1-GFP of 1.5 × 10⁶ PFU was added to 5 × 10⁴ CHO-K1 cells to which human PILR had been transfected, and expression of GFP was analyzed 18 hours later.

30 minutes before adding the virus, 100µM or 500µM of acyclovir, or 10µg/ml of anti-human PILR mAb (M4) was added, and a ratio of infection with the virus was shown in Fig. 10 with a ratio of infection of cells to which nothing had been added ("control" in Fig. 10) being 100%.

As shown in Fig. 10, it was confirmed that adding a sufficient amount of acyclovir that was an anti-HSV drug did not inhibit infection with HSV, but adding an anti-PILR antibody inhibited the infection. That is, data in Fig. 10 shows that acyclovir terminates the infected cells, but cannot inhibit the infection itself.

The herpes virus infection inhibitor of the present invention contains an effective component capable of binding to glycoprotein B or a receptor for glycoprotein B and capable of inhibiting interaction between glycoprotein B and the receptor for glycoprotein B. Therefore, the herpes virus infection inhibitor of the present invention can prevent infection of a host with herpes virus, regardless of whether the effective component is an anti-PILR antibody or not or whether the effective component is soluble PILR or not.

The invention being thus described, it will be obvious that the same way may be varied in many ways. Such variations are not to be regarded as a departure from the spirit and scope of the invention, and all such modifications as would be obvious to one skilled in the art are intended to be included within the scope of the following claims.

### Industrial Applicability

The herpes virus infection inhibitor of the present invention is preferably applicable to a medicine for preventing infection with herpes virus. Since the herpes virus infection inhibitor has an action mechanism different from that of acyclovir that is a known anti-herpes virus drug, the herpes virus infection inhibitor can be used as a herpes virus infection therapeutic drug singularly or in combination with acyclovir. Therefore, the herpes virus infection inhibitor of the present invention has very high industrial applicability in the field of medicine industries etc.

## Claims

1. A herpes virus infection inhibitor, containing an effective component capable of binding to glycoprotein B or a receptor for glycoprotein B and capable of inhibiting interaction between glycoprotein B and the receptor for glycoprotein B.

2. The herpes virus infection inhibitor as set forth in claim 1, wherein the effective component is capable of binding to PILR that is a receptor for glycoprotein B and capable of inhibiting interaction between PILR and glycoprotein B.

3. The herpes virus infection inhibitor as set forth in claim 2, wherein the effective component is an anti-PILR antibody.

4. The herpes virus infection inhibitor as set forth in claim 1, wherein the effective component is capable of binding to glycoprotein B and capable of inhibiting interaction between a receptor for glycoprotein B and glycoprotein B.

5. The herpes virus infection inhibitor as set forth in claim 4, wherein the effective component is soluble PILR.

6. A method for inhibiting infection with herpes virus, comprising the step of administering a herpes virus infection inhibitor as set forth in any one of claims 1 to 5 to a host and inhibiting interaction between a receptor for glycoprotein B and glycoprotein B in the host, so that infection of the host with herpes virus is inhibited.

7. A receptor for herpes virus, made of PILR and capable of binding to glycoprotein B contained in the herpes virus.

8. An animal or a cell susceptible to herpes virus, containing a receptor as set forth in claim 7.
